# EUROPEAN PATENT SPECIFICATION

(11) **EP 0 448 336 B1**
(45) Date of publication and mention of the grant of the patent: **20.07.1994**
(21) Application number: 91302336.2
(22) Date of filing: 19.03.1991
(51) Int. Cl.: A61G 7/05, A61F 5/01

(54) **Cushion**
Kissen
Coussin

(30) Priority: 19.03.1990 GB 9006180
(43) Date of publication of application: 25.09.1991
(73) Proprietor: U.T. Care Products Limited, London (GB)
(72) Inventor: Swain, Ian Douglas, Nr. Salisbury SP2 9NR (GB); Lowthian, Peter Thomas, Watford WD1 4AJ (GB)
(74) Representative: Charlton, Peter John

(56) References cited:
- BE-A- 352 256
- DE-A- 1 457 483
- DE-C- 47 394
- US-A- 3 276 047
- US-A- 3 935 604
- US-A- 4 643 481
- US-A- 4 824 174

## Description

The present invention relates to a seat cushion, in particular a cushion for use by patients, for example for the relief of pain associated with perineal wounds and similar problems.

A great number of cushions have been designed for medical use, for example ones being essentially ring-shaped and ones being formed of sculptured foam with specially shaped recesses. There is also known from US-A-3276047 a cushion formed of two inflatable bags with a parallel-sided valley therebetween. However, there remains a need for a cushion which can be comfortable for patients for long periods of time and which is sufficiently versatile to be used by patients of different builds and suffering from different conditions.

An object of the invention is therefore to provide a new and improved support seat cushion.

According to present invention there is provided a seat cushion comprising a pair on inflatable bags, each bag being arranged laterally with respect to the other to define a channel between the bags, characterised in that the channel is tapered along its length from one side of the cushion to an opposite side of the cushion.

Advantageously, each bag has means to form a depression in the bag in its inflated state, the depression running along the bag substantially in the same direction as the channel.

Preferably, each bag has means to form a depression on both its upper and lower surfaces in its inflated state. The two depressions are preferably set at slightly different distances from the channel.

Preferably, the means to form the depressions consist of elongate pads, the pads being shaped so that when the bags are inflated they press into the bags. The pads may be arranged on the bags in a slightly convex orientation, each pad curving away from the channel.

In a preferred embodiment, an additional pad (preferably inflatable) is provided for the cushion, this pad being able to adopt different positions along the length of the channel.

For the reasons given below, the cushion of the invention is extremely comfortable for patients and also very versatile. The inflatable bags and the tapered channel provide for a variety of different support patterns and positions of use.

The purpose of the channel of the cushion is to relieve pressure when the user is seated. Normally, this pressure relief is from the perineum and the "perianal" areas of the body, the person using the cushion with the central channel running from back to front (rather than from side to side).

The central channel or valley of the cushion is tapered so that it can be used by a wide range of differently sized individuals. This is because, when sitting upright, a person's ischial tuberosities bear most of his or her trunk weight, and these bony prominences vary in their width of separation (from about 8 to 17 cms.). However, when sitting upright, these bony prominences are always about 6 to 15 cms. from the rear edge of the cushion. In consequence, in one fore and aft orientation, the cushion will suit a person with widely-spaced prominences (Ischia) whereas, when turned through 180 degrees, it will suit a person with narrowly-spaced ischia. The channel is, in fact, shaped so as to allow most of a person's weight to be supported on the outer aspect of each ischial tuberosity and on his "Trochanteric shelf" (hip bone prominences) when it is used in the fore and aft position. On the relatively rare occasions when the new cushion is found more comfortable with the channel placed side-to-side, the main weight of the user will be taken on the great tendons of the hamstring muscles (just in front of the ischia) and, to some degree, on the broad (Sacrotuberous) ligaments stretched between the ischia and the sacrum.

Because the main supportive elements of the cushion, on either side of the central channel, are inflatable, the actual pressures on the various parts of the person's posterior, can be individually adjusted. The inflatable bags, can also be inflated to different levels of pressure, or to the same level. The facility for asymmetric inflation will be particularly useful for persons with unusual pelvic anatomy.

The purpose of placing the elongate pads at slightly different distances apart on each side of the cushion, is that the cushion gives a greater range of channel widths - to suit a greater number of people - depending on which way up it is used. Moreover, on one side of the cushion the channel is slightly more prominent than on the other. The shallow channel side is designed to suit those who need a relatively firm cushion with only minimal central pressure relief at low inflation levels.

A principal use for the cushion may be for women who have just given birth, and have had either an accidental tear of the perineum, or a repaired episiotomy wound. However it may also be useful, in the same back to front orientation, for persons (of either sex) suffering from painful haemorrhoids (piles) or surgical wounds on, or near, the anus. Fracture of the coccyx, and similar painful conditions would also benefit from the use of the cushion.

Another use for the cushion may be amongst patients with threatened pressure sores, of the coccyx or "Sacral" areas, these being particularly common in elderly, hospitalised, patients. Pressure areas on the buttocks (Ischial tuberosities) will also benefit from the new cushion. Such sores often occur in patients undergoing cardiac surgery. In this latter application the central channel may give more relief if it runs "side to side" - under the ischial tuberosities.

In all of the above-mentioned applications, the particular benefit of the cushion over traditional cushions like rings, is that the person's blood circulation is not compromised by circumferential pressure on the soft tissues. Also the tired or debilitated person's natural inclination to slide forwards when seated for long periods is more safely accommodated than if he/she were on a ring cushion. Further, the elongate pads, in the preferred version of the cushion, prevent discomfort from pressure being present on nerves (neurapraxia) when the cushion is in use for long periods - as will be discussed below.

As mentioned above, a small auxiliary pad to be used in any position along the central channel of the cushion will be available. This will allow moderate pressure to be applied, along the central channel, wherever this is needed. In particular, this may provide the correct level of pressure to support painful haemorrhoids.

A preferred embodiment of the present invention will now be described in more detail, by example only, and with reference to the accompanying drawings, wherein:
Fig. 1 is a plan view of the cushion according to this embodiment, without any cover thereon;
Figs. 2a and 2b are schematic cross-sectional views of the cushion with the bags in two different states of inflation, the inflated bag having only one elongate pad and the left bag being shown with no pad for the purposes of comparison; and
Fig. 3 is a detail showing one of the elongate pads before it is fitted to the cushion.

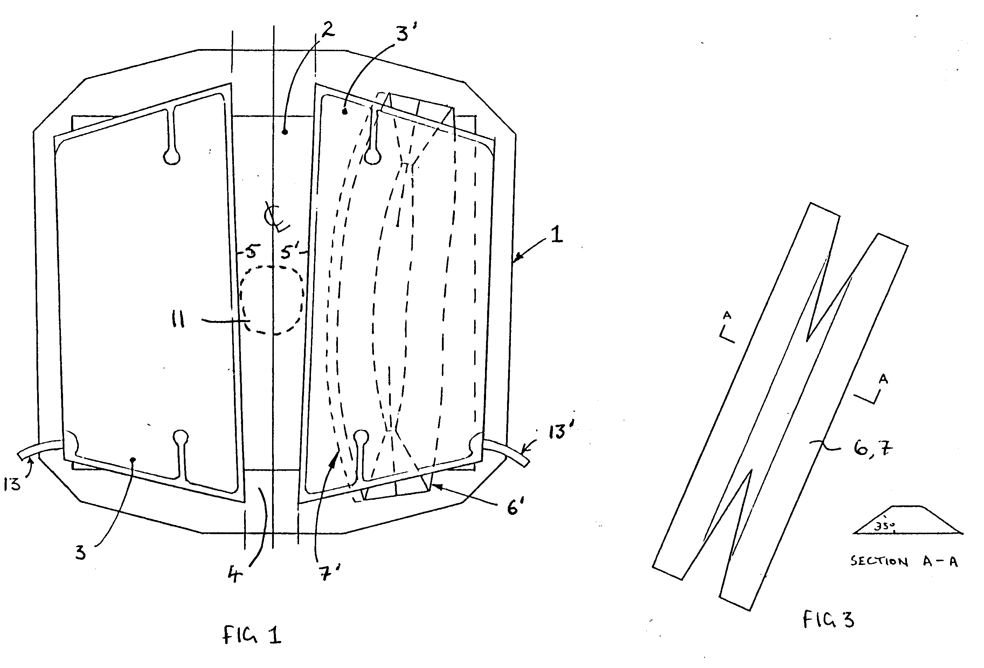

The cushion shown in Fig. 1 comprises a 20 mm thick foam base sheet 1 on which is bonded a woven-nylon connecting sheet 2. Two rubber inflatable bags 3,3′ are fitted on the sheet 2, as described below, to define a tapering channel 4 therebetween. The bags are generally trapezoidal in shape; the dimensions may be 390 mm by 170 mm, with the largest dimension being adjacent the channel 4. A high pressure valve 13,13 is fitted at the corner of each bag.

The inner edges 5,5′ of the bags 3,3′ are bonded to the sheet 2 to define boundaries of the channel 4, which may taper from 80mm to 50mm, for example. The widest width can vary from 40mm to 150mm and the narrower width from 20mm to 100mm. The wider end is preferably at least 20mm wider than the narrower end. The taper is thus preferably at least 1 in 40. It will be appreciated that the channel is trapezoidal in shape, rather than triangular.

Lower, elongate pads 6,6′ are then bonded to the sheet 2, beneath the bags 3,3′. The lower surfaces of the bags 3,3′ are then bonded to the pads 6,6′ and the sheet 2.

Upper elongate pads 7, 7′ are bonded on top of the bags 3,3′.

It will be seen that the pads 6,6′,7,7′ are arranged so that they extend generally parallel to the channel 4, but the upper pads 7,7′ are nearer to the channel.

The pads are glued in such a position that they are slightly curved.

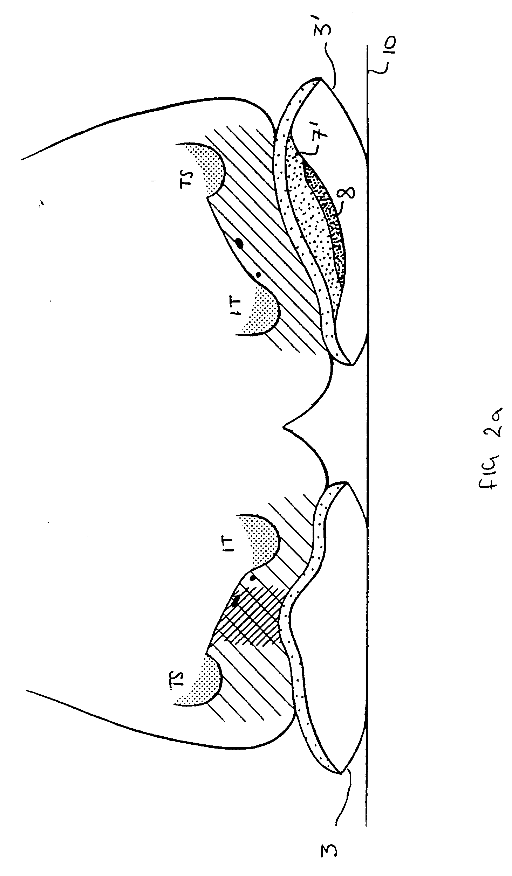

Fig. 2a shows in section a person seated on the cushion which in turn rests on a hard flat surface 10. The left hand inflated bag 3 is not fitted with an elongate pad, but the right hand bag 3′ does have an elongate pad 7′ on its upper surface.

The person's Trochanteric Shelves and Ischial Tuberosities which are discussed above are indicated by letters TS and IT respectively. Low pressure zones within the person's buttocks are indicated by broad hatching and high pressure zones are indicated by narrow hatching. The localization of these high pressure zones becomes more apparent after one or two hours of sitting.

It can clearly be seen that the pad 7′ which forms a depression in the bag 3′ prevents a high pressure area developing between Trochanteric Shelf and the Ischial Tuberosity (which in fact is where the sciatic nerve is found). The high pressure area is seen in the left buttock where the bag without the pad is forced to "bulge" out between the TS and the IT.

Fig. 2b shows a section view which is similar to Fig. 2a, but which the inflatable bags 3,3′ inflated to a higher level. However, much the same reduction in pressure in the buttocks is achieved with the bag 3′ having the pad 7 as with the bag in Fig. 2a. The high pressure area formed by the bag 3 without the pad is slightly more central and in fact affects the post-cutaneous nerve in addition to the sciatic nerve.

Needless to say, the avoidance of high pressure areas provides much greater comfort during prolonged use of the cushion.

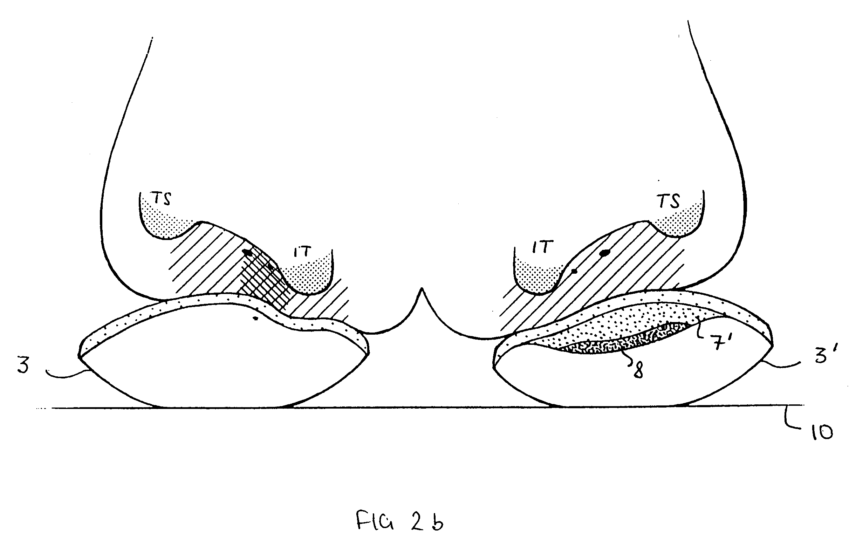

As more clearly seen in Fig 3, the pads are trapezoidal in cross-section. This tapering or pointed shape is preferred so that the pads, when the bags are inflated, more clearly form depressions or indents along the length of the bags. For this purpose, the pads are glued in position with their narrower dimension next to the bags. Moreover, in a preferred refinement, the lower part of each pad is made in relatively inflexible material (8 in Fig. 2a and 2b) to encourage full-length depressions to form in the bags.

In order that the pads fit smoothly on the cushion, their ends are chamfered. This may be achieved by cutting "V" notches into the ends of lengths of the trapezoidally sectioned foam material. The limbs of the "V"'s are then glued together when the pads are bonded to the bags.

Two strips of "Velcro" (Registered Trade Mark) are bonded to the underside of the auxiliary pad 11 (shown in dotted lines in Fig. 1) to allow its temporary attachment to the cushion's central channel area in a variety of different positions; the central channel area is covered with a suitable "Velcro" attachment fabric.

The inflation bags are made from extremely strong synthetic material, as used for blood pressure measuring cuffs. The valves used are designed to retain air pressures, as in car tyres, for long periods. The bags may be inflated by mouth or more preferably by means of a hand pump, as used for blood pressure cuff inflation, for example.

The overall shape of the cushion is designed to be suitable for use on either bed or chair, as well as to make the correct orientation of the cushion obvious.

The foam parts of the cushion are chosen to be both comfortable and safe. The foam is flame-retardant to comply with the relevant legislation.

The outer cover is waterproof, and can be wiped down when soiled. It is open at each side, so as to provide good ventilation. At the same time, the openings are extended beyond the cushion edges, so that they drop downwards (and should be tucked under each side of the cushion). Thus there is little chance of fluid getting inside the cushion. Should this happen, however, the cover can be washed - at low temperature

## Claims

1. A seat cushion comprising a pair of inflatable bags (3,3′), each bag being arranged laterally with respect to the other to define a channel (4) between the bags, characterised in that the channel (4) is tapered along its length from one side of the cushion to an opposite side of the cushion.

2. A seat cushion according to claim 1, wherein each bag (3,3′) has means (6,6′;7,7′) to form a depression in the bag in its inflated state, the depression running along the bag substantially in the same direction as the channel (4).

3. A seat cushion according to claim 2, wherein each bag (3,3′) has means (6,6′;7,7′) to form a depression on both its upper and lower surfaces in its inflated state.

4. A seat cushion according to claim 3, wherein two depressions of each bag are set at slightly different distances from the channel.

5. A seat cushion according to claim 2, 3 or 4, wherein the means to form the depressions consist of elongate pads (6,6′;7,7′), the pads being shaped so that when the bags (3,3′) are inflated they press into the bags.

6. A seat cushion according to claim 5, wherein the pads (6,6′;7,7′) are arranged on the bags (3,3′) in a slightly convex orientation, each pad curving away from the channel.

7. A seat cushion according to any preceding claim further comprising an additional pad (11), this pad being able to adopt different positions along the length of the channel (4).

8. A seat cushion according to claim 7, wherein the additional pad (11) is inflatable.

## Patentansprüche

1. Sitzkissen umfassend ein Paar aufblasbare Kammern (3, 3′), wobei jede Kammer in Bezug auf die andere seitlich angeordnet ist, so daß ein Kanal (4) zwischen den Kammern definiert ist, dadurch gekennzeichnet, daß der Kanal (4) entlang seiner Länge sich von einer Seite des Kissens zu einer gegenüberliegenden Seite des Kissens verjüngt.

2. Sitzkissen nach Anspruch 1, worin jede Kammer (3,3′) Mittel (6,6′;7,7′) aufweist, so daß in der Kammer im aufgeblasenen Zustand eine Vertiefung gebildet ist, wobei die Vertiefung entlang der Kammer im wesentlichen in derselben Richtung verläuft wie der Kanal (4).

3. Sitzkissen nach Anspruch 2, worin jede Kammer (3,3′) Mittel (6,6′;7,7′) aufweist, so daß sowohl auf ihrer Ober- wie auf ihrer Unterseite im aufgeblasenen Zustand eine Vertiefung gebildet ist.

4. Sitzkissen nach Anspruch 3, worin zwei Vertiefungen jeder Kammer in etwas unterschiedlichen Abständen vom Kanal angeordnet sind.

5. Sitzkissen nach Anspruch 2, 3 oder 4, worin die Mittel zum Ausbilden der Vertiefungen aus langgestreckten Polstern (6,6′;7,7′) bestehen, wobei die Polster so geformt sind, daß sie in die Kammern drücken, wenn die Kammern (3,3′) aufgeblasen sind.

6. Sitzkissen nach Anspruch 5, worin die Polster (6,6′; 7,7′) auf den Kammern (3,3′) in einer etwas konvexen Orientierung angeordnet sind, wobei jedes Polster vom Kanal weg gewölbt ist.

7. Sitzkissen nach einem der vorhergehenden Ansprüche ferner umfassend ein zusätzliches Polster (11), wobei dieses Polster fähig ist, entlang der Länge des Kanals (4) unterschiedliche Positionen einzunehmen.

8. Sitzkissen nach Anspruch 7, worin das zusätzliche Polster (11) aufblasbar ist.

## Revendications

1. Un coussin pour siège comprenant une paire de sacs gonflables (3,3′), chaque sac étant disposé latéralement l'un par rapport à l'autre de sorte à former un canal (4) entre les sacs, caractérisé en ce que le canal (4) est effilé longitudinalement depuis un côté du coussin jusqu'à un côté opposé du coussin.

2. Un coussin pour siège selon la Revendication 1, dans lequel chaque sac (3,3′) est pourvu de moyens (6,6′; 7,7′) pour former une dépression dans le sac lorsqu'il est à l'état gonflé, la dépression s'étendant le long du sac substantiellement dans la même direction que le canal (4).

3. Un coussin pour siège selon la Revendication 2, dans lequel chaque sac (3,3′) est pourvu de moyens (6,6′; 7,7′) pour former une dépression sur les surfaces supérieure et inférieure lorsqu'il est à l'état gonflé.

4. Un coussin pour siège selon la Revendication 3, dans lequel deux dépressions de chaque sac sont disposées à des distances légèrement différentes du canal.

5. Un coussin pour siège selon l'une des Revendications 2, 3 ou 4, dans lequel les moyens permettant de former les dépressions se composent de patins allongés (6,6′; 7,7′), les patins étant conçus de sorte à exercer une pression à l'intérieur des sacs lorsque les sacs (3,3′) sont à l'état gonflé.

6. Un coussin pour siège selon la Revendication 5, dans lequel les patins (6,6′; 7,7′) sont disposés sur les sacs (3,3′) selon une orientation légèrement convexe, chaque patin se cintrant du côté opposé au canal.

7. Un coussin pour siège selon l'une quelconque des Revendications précédentes, comprenant en outre un patin supplémentaire (11), ce patin étant capable d'adopter différentes positions sur la longueur du canal (4).

8. Un coussin pour siège selon la Revendication 7, dans lequel le patin supplémentaire (11) est gonflable.
